# EUROPEAN PATENT APPLICATION

(11) **EP 3 696 282 A1**
(43) Date of publication of application: **19.08.2020**
(21) Application number: 19382103.0
(22) Date of filing: 14.02.2019
(51) Int. Cl.: C12Q 1/6883

(54) **METHOD FOR DIAGNOSING UNSTABLE ATHEROSCLEROTIC PLAQUE**

(71) Applicant: Universidad del Pais Vasco, 48940 Leioa Vizcaya (ES); Administración General de la Comunidad Autónoma de Euskadi, 01010 Vitoria-Gasteiz (ES)
(72) Inventor: ALLOZA MORAL, Iraide, 48170 Zamudio, Bizkaia (ES); VANDENBROECK, Koen, 48940 Leioa, Vizcaya (ES); FREIJO GUERRERO, María del Mar, 48013 Bilbao (ES); VEGA MANRIQUE, Reyes, 48013 Bilbao (ES)
(74) Representative: ABG Intellectual Property Law, S.L.

(57) **Abstract**

The present invention relates to a diagnostic method for diagnosing unstable atherosclerotic plaque, a method for determining the probability of a subject suffering from a cerebrovascular disease, and a method for selecting a patient susceptible to being treated with a therapy for removing or stabilizing the carotid atherosclerotic plaque based on determining the allele in the sequence of a SNP. The invention also relates to a kit comprising reagents suitable for determining the allele in the sequence of said SNP, and optionally reagents for determining the expression levels of one or more genes, and uses of said kit.

## Description

### TECHNICAL FIELD OF THE INVENTION

The present invention relates to a diagnostic method for diagnosing unstable atherosclerotic plaque, to a method for determining the probability of a subject suffering from a cerebrovascular disease and to a method for selecting a patient susceptible of being treated with a therapy for removing or stabilizing the carotid atherosclerotic plaque.

### BACKGROUND OF THE INVENTION

Cerebrovascular disease is one of the main causes of death and disabilities in developed societies. The risk of suffering from a cerebrovascular accident (CVA) doubles each decade after 55 years of age. Though it may occur at any age, three quarters of CVAs occur in people over 65 years of age. Taking into account the progressive aging of the European population, this disease will be a major health issue in the near future. Specifically, the World Health Organization (WHO) already considers strokes, which are a type of CVA, to be one of the most serious health issues Europe is facing.

Stenosis caused by the formation of atherosclerotic plaque in the carotid artery is one of the main factors for developing a cerebrovascular disease. The plaque can even become unstable and cause a cerebrovascular accident. The process whereby plaque becomes unstable is not a random process and there is evidence that it is a biological process, although the precise mechanism whereby destabilization of the atherosclerotic plaque takes place is unknown. The instability of the atheromatous plaque of the carotid artery is therefore a risk factor for the development of a CVA, even if it is not always associated with the presence of diagnosable symptoms. Atherosclerotic plaques can be defined as stable or asymptomatic when they are made up of a small lipid core and a stable fibrous plaque, or as symptomatic when they are made up of a large lipid core and a thin fibrous plaque with the propensity to become detached. An unstable plaque is related to a higher risk of developing a CVA.

Due to the complexity of the processes that trigger a CVA, the difficulty in finding biomarkers with a real diagnostic value is known in the state of the art (Jickling et al., Stroke 46, 915-920, 2015), and there are no biomarker-based tests that help to identify stable or unstable plaques or to assess the risk of developing a CVA (Hermus et al., Atherosclerosis 213, 21-29, 2010).

There is therefore a need to identify biomarkers with a diagnostic value that allow identifying patients with unstable atherosclerotic plaques and therefore with a higher risk of suffering from a cerebrovascular disease.

### SUMMARY OF THE INVENTION

In a first aspect the invention relates to a method for diagnosing unstable atherosclerotic plaques in a subject which comprises determining the SNP having accession number rs35286811 in a biological sample from said subject, and diagnosing unstable atherosclerotic plaque in the subject according to the sequence of said SNP.

In a second aspect, the invention relates to a prognostic method for determining the probability that a subject develops a cerebrovascular disease which comprises determining the SNP having accession number rs35286811 in a biological sample from said subject, and predicting the probability that the subjects develops a cerebrovascular disease according to the sequence of said SNP.

In a third aspect, the invention relates to a method for selecting a patient susceptible of being treated with a therapy for removing or stabilizing the carotid atherosclerotic plaque which comprises determining the SNP having accession number rs35286811 in a biological sample from said patient, and selecting the patient as susceptible to being treated for removing or stabilizing the carotid atherosclerotic plaque according to the sequence of said SNP.

In a fourth aspect, the invention relates to a kit comprising reagents suitable for determining the allele in the sequence of the SNP having accession number rs35286811.

### DETAILED DESCRIPTION OF THE INVENTION

The authors of the present invention have identified that the alleles in the SNP having accession number rs35286811 differ between samples obtained from patients diagnosed with symptomatic carotid plaques with respect to patients with asymptomatic plaques (Example 1). Accordingly, the alteration in the nucleotide of at least one allele of said SNP can be used for diagnosing the stability or instability of an atherosclerotic plaque based on a biological sample from a subject.

### Method for diagnosing unstable atherosclerotic plaques

In a first aspect the invention relates to a method for diagnosing unstable atherosclerotic plaques in a subject which comprises determining the SNP having accession number rs35286811 in a biological sample from said subject, and diagnosing unstable atherosclerotic plaque in the subject according to the sequence of said SNP.

As it is used herein, "diagnostic method" or "diagnose" refers both to the process of trying to determine and/or identify a possible disease in a subject, i.e., the diagnostic process, and to the opinion that is arrived at by means of this process, i.e., the diagnostic opinion. As such, it can also be considered an attempt to classify the state of an individual in different and separate categories that allow making medical decisions concerning the treatment and prognosis. As those skilled in the art will understand, the diagnosis of unstable atherosclerotic plaque, is not necessarily, although it is preferably, correct for 100% of the subjects to be diagnosed or evaluated. However, the term requires enabling identification of a statistically significant part of the subjects who have an unstable atherosclerotic plaque. One skilled in the art can readily determine if a subject is statistically significant by using various well-known statistical evaluation tools, for example, the determination of confidence intervals, the determination of the p-value, Student's t-test, Mann-Whitney test, etc. Details can be found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are preferably 0.05, 0.01, 0.005, or lower.

As it is used herein, the term "atherosclerosis" refers to a pathology characterized by the deposition and infiltration of lipid substances in the walls of thick medium-sized arteries. The cells of the arterial wall interpret this deposition as an invasion and activate circulating monocytes of the immune system, which penetrate the arterial wall, turn into macrophages and begin to phagocyte LDL particles, generating an inflammatory process. The inflammation in turn causes the multiplication and migration of the smooth muscle cells of the wall, which causes a gradual stricture of the diameter of the artery. The specific thickening constitutes an atheromatous plaque. Included among those diseases presenting with atherosclerosis characterized by the stricture of the arteries due to atheromatous plaques, and accordingly obstruction of the blood flow or ischemia, depending on the artery of the organ involved, are: ischemic heart disease (with acute myocardial infarction of the heart being the maximum representative), cerebrovascular disease (in the form of stroke or cerebral thrombosis or intracerebral hemorrhage, in the central nervous system), intermittent claudication (with the most serious form being arterial ischemia of the lower extremities), erectile dysfunction, ischemic colitis (an area of inflammation, irritation, and swelling, caused by interference with the blood flow in the colon, in the arteries of the intestine), and aortic aneurism.

As it is used herein, "atherosclerotic plaque," also known in the art as "atheromatous plaque," refers to the buildup of lipid substances on arterial walls according to the definition of "atherosclerosis" until forming a plaque formed by lipids, macrophages, and smooth muscle cells. The atherosclerotic plaque can be stable or unstable.

The term "stable plaque," also known in the art as "stable atherosclerotic plaque" or "asymptomatic plaque," has been used to describe atherosclerotic plaques that are not particularly susceptible to rupture. They are generally characterized by having a thick fibrous cap over a small lipid core.

The term "unstable atherosclerotic plaque," also known in the art as "unstable plaque" or "symptomatic plaque," has been used to describe atherosclerotic plaques that are particularly susceptible to rupture. They are generally characterized as having an inflamed thin fibrous cap over a very large lipid core. It consists of a mixture of white blood cells (primarily macrophages), smooth muscle cells (SMCs), and lipids (including cholesterol) in the wall of an artery. They are particularly unstable and have the propensity to cause sudden problems, such as a heart attack or stroke. The characteristics that define an unstable plaque include: inflammatory activity, a thin fibrous cap, a large lipid core, visible intra-plaque ulceration, bleeding, and rupture.

In a particular embodiment of the methods of the invention, the unstable atherosclerotic plaque is a carotid plaque. The blood supply to the carotid artery begins in the aortic arch; the carotid artery is divided into the internal carotid artery (which supplies blood to the brain) and the external carotid artery. The atherosclerotic plaque usually builds up in that division of the carotid artery, which causes stricture, also known as stenosis.

The term "subject" or "individual" or "patient" refers to any subject, particularly a mammalian subject, for whom the diagnosis, prognosis, or treatment is desired. Mammalian subjects include human beings, domestic animals, farm animals, and zoo animals, animals used for sports, or domestic animals such as dogs, cats, guinea pigs, rabbits, rats, mice, horses, cattle, cows, and so on and so forth. In a preferred embodiment of the invention, the subject is a mammal. In a more preferred embodiment of the invention, the subject is a human being. In another particular embodiment, the subject is a subject who potentially has a disease associated with an alteration in atherosclerotic plaques.

In a particular embodiment, the subject whose diagnosis is to be determined according to the first method of the invention has already had a cerebrovascular complication prior to being diagnosed with unstable atherosclerotic plaque. In a particular embodiment, said cerebrovascular complication is selected from the group consisting of a transient ischemic attack (TIA), stroke, and amaurosis fugax.

According to the methods of the invention, the SNP having accession number rs35286811 is determined in a biological sample from the subject.

The term "single nucleotide polymorphism" or "SNP", as used herein, refers to a variation in the nucleotide sequence of a nucleic acid that occurs in a single nucleotide (A, C, T or G), wherein each possible sequence is present in a proportion equal to or greater than a 1% of the population. The SNPs are typically named using the accession number in the SNP database (dbSNP) at National Center for Biotechnology Information (NCBI) accessible at www.ncbi.nlm.nih.gov/projects/SNP/. In general, SNPs represent one of the most common forms of genetic variations. These polymorphisms appear when a single nucleotide in the genome is altered (such as via substitution, addition or deletion). Each version of the sequence with respect to the polymorphic site is referred to as an allele of the polymorphic site. SNPs tend to be evolutionary stable from generation to generation and, as such, can be used to study specific genetic abnormalities throughout a population. If SNPs occur in the protein coding region, it can lead to the expression of a variant, sometimes defective, form of the protein that may lead to development of a genetic disease. Some SNPs may occur in non-coding regions, but nevertheless, may result in differential or defective splicing, or altered protein expression levels. SNPs can therefore serve as effective indicators of a genetic disease. SNPs can also be used as diagnostic and/or prognostic tools for identifying individuals with a predisposition for a disease or for a fast evolution of the disease, genotyping the individual suffering from the disease, and facilitating drug development based on the insight revealed regarding the role of target proteins in the pathogenesis process. Each version of the sequence with respect to the SNP is referred to as an allele of the SNP.

The term "allele", as used herein, relates to one of two or more forms of a gene, locus or genetic polymorphism. Sometimes, different alleles can result in different traits; however, other times, different alleles will have the same result in the expression of a gene. Most multicellular organisms have two sets of chromosomes, that is, they are diploid. These chromosomes are referred to as homologous chromosomes. Diploid organisms have one copy of each gene (and one allele) on each chromosome. If both alleles are the same, they are homozygotes. If the alleles are different, they are heterozygotes.

The terms "determining the SNP" or "detecting an SNP" are used indistinctly in the present invention, and refer to the determination of the sequence of a particular SNP in any one or both alleles of the subject under study. The determination of the sequence of the SNP can be performed by means of multiple processes known by the person skilled in the art. In a particular embodiment the determination of the sequence of the SNPs can be carried out in a sample from whole blood, and it may be used directly for the detection of said SNPs. In other embodiments, the nucleic acid can be extracted from cells which are present in a biological fluid (e.g., whole blood, saliva, synovial fluid, etc.) as an initial step, and, in such cases, the total nucleic acid extracted from said samples would represent the working material suitable for subsequent amplification. Isolating the nucleic acid of the sample can be performed by methods known by the person skilled in the art. Said methods can be found, for example, in Sambrook et al., 2001. "Molecular cloning: a Laboratory Manual", 3rd ed., Cold Spring Harbor Laboratory Press, N.Y., Vol. 1-3. Further, as mentioned above, in some embodiments, generation of nucleic acids for analysis from samples requires nucleic acid amplification. Many amplification methods rely on an enzymatic chain reaction such as, for example, a polymerase chain reaction (PCR), a ligase chain reaction (LCR), or a self-sustained sequence replication, rolling-circle amplification assays, etc.; this list is merely illustrative and in no way limiting. Methods for amplifying nucleic acid are described in Sambrook et al., 2001 (cited at supra).

The presence or absence of the SNP can be identified by amplifying or failing to amplify an amplification product from the sample. Polynucleotide amplifications are typically template-dependent. Such amplifications generally rely on the existence of a template strand to make additional copies of the template. Primers are short nucleic acids that are capable of priming the synthesis of a nascent nucleic acid in a template-dependent process, which hybridize to the template strand. Typically, primers are from ten to thirty base pairs in length, but longer sequences can be employed. Primers may be provided in double-stranded and/or single-stranded form, although the single-stranded form generally is preferred. Often, pairs of primers are designed to selectively hybridize to distinct regions of a template nucleic acid, and are contacted with the template DNA under conditions that permit selective hybridization. Depending upon the desired application, high stringency hybridization conditions may be selected that will only allow hybridization to sequences that are completely complementary to the primers. Hybridization may also occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced. The nucleotides present in the polymorphic markers can be determined from either nucleic acid strand or from both strands. Any of a variety of methods that exist for detecting sequence variations may be used in the methods of the invention.

After isolating, and amplifying (if necessary), the nucleic acid, the sequences of the different SNPs of the invention are detected. Those skilled in the art will readily recognize that the analysis of the nucleotides present in one or several of the SNPs, or polymorphisms, disclosed herein in a patient's nucleic acid can be done by any method or technique capable of determining nucleotides present in a SNP or polymorphism. For instance, one may detect the SNP in the first method of the invention by performing sequencing, mini-sequencing, hybridization, restriction fragment analysis, oligonucleotide ligation assay, allele-specific PCR, or a combination thereof. As such, systems and methods for the detection of SNPs, in general include, but are not limited to, nucleic acid sequencing, hybridization methods and array technology (e.g. technology available from Aclara BioSciences, Affymetrix, Agilent Technologies, Illumina Inc., etc); also techniques based on mobility shift in amplified nucleic acid fragments, Single Stranded Conformational Polymorphism (SSCP), denaturing gradient gel electrophoresis (DGGE), Chemical Mismatch Cleavage (CMC), Restriction Fragment Polymorphisms (RFLPs), and WAVE analysis can be used (Methods Mol. Med. 2004; 108: 173-88), real-time PCR, and the like. Of course, this list is merely illustrative and in no way limiting. Those skilled in the art may use any appropriate method to achieve such detection. As it is obvious in the art, the sequence of said SNPs can be determined from any one of the nucleic acid strands or from both strands.

A variation on the direct sequence determination method is the Gene Chip(TM) method available from Affymetrix. Other methods include: Perkin Elmer adapted its TAQman Assay(TM) to detect sequence variation. Orchid BioSciences has a method called SNP- IT (TM). (SNP-Identification Technology) that uses primer extension with labeled nucleotide analogs to determine which nucleotide occurs at the position immediately 3' of an oligonucleotide probe, the extended base is then identified using direct fluorescence, indirect colorimetric assay, mass spectrometry, or fluorescence polarization. Sequenom uses a hybridization capture technology plus MALDI-TOF (Matrix Assisted Laser Desorption/Ionization--Time-of-Flight mass spectrometry) to detect SNP genotypes with their MassARRAY(TM) system. Promega provides the READIT(TM) SNP/Genotyping System (U.S. Pat. No. 6,159,693). In this method, DNA or RNA probes are hybridized to target nucleic acid sequences. Probes that are complementary to the target sequence at each base are depolymerized with a proprietary mixture of enzymes, while probes which differ from the target at the interrogation position remain intact. The method uses pyrophosphorylation chemistry in combination with luciferase detection to provide a highly sensitive and adaptable SNP scoring system. Third Wave Technologies has the Invader OS(TM) method that uses a proprietary Cleavaseg enzymes, which recognize and cut only the specific structure formed during the Invader process. Invader OS relies on linear amplification of the signal generated by the Invader process, rather than on exponential amplification of the target. The Invader OS assay does not utilize PCR in any part of the assay. In addition, there are a number of forensic DNA testing labs and many research labs that use gene-specific PCR, followed by restriction endonuclease digestion and gel electrophoresis (or other size separation technology) to detect restriction fragment length polymorphisms (RFLPs).

Any other commercially available method can also be used for determining a SNP, by the high throughput SNP identification not using direct sequencing technologies. Non-limiting examples include: Illumina's Veracode Technology, Taqman® SNP Genotyping Chemistry, KASPar SNP genotyping Chemistry and Fluidigm SNP genotyping.

According to the invention, the SNP can be detected in any nucleic acid. "Nucleic acid" as used herein mean biopolymers of nucleotides, which are linked with one another via phosphodiester bonds (polynucleotides, nucleic acids). Nucleic acids may additionally be linked via phosphorothioate bonds when chemically synthesized. Depending on the type of sugar in the nucleotides (ribose or deoxyribose), one distinguishes the two classes of the ribonucleic acids (RNA) and the deoxyribonucleic acids (DNA). As used herein it relates to any natural or non-natural nucleic acid and "oligonucleotide" and "polynucleotide" are used interchangeably in the context of the present invention. A nucleic acid sequence is represented in 5'->3' direction unless indicated otherwise. In one embodiment, the nucleic acid used to determine the presence of the SNP is RNA. In another embodiment, the nucleic acid in which the presence or absence of the SNP is determined is DNA.

In a first step, the diagnostic method of the invention comprises determining the SNP having accession number rs35286811.

The SNP rs35286811 is located in the BIRC6 gene. "BIRC6" " refers to the Baculoviral IAP repeat-containing protein 6, which in humans has the sequence shown in the Uniprot database under accession number Q9NR09, entry version 170 (date 5/12/2018).

According to the invention, the SNP consists of two alleles (G and C). The SNP can be detected in any sample containing any kind of nucleic acid. In a preferred embodiment, the SNP is detected in RNA and/or in DNA isolated from a sample. In a more preferred embodiment, the SNP is detected in RNA. In a still more preferred embodiment, the SNP is detected in DNA, preferably genomic DNA. In a preferred embodiment if a person carries the C allele of the SNP rs35286811 on one chromosome (heterozygote), then the subject is diagnosed with unstable atherosclerotic plaque. In a more preferred embodiment if a person carries the C allele of the SNP rs35286811 on both chromosomes (homozygote), then the subject is diagnosed with unstable atherosclerotic plaque.

The term "sample" or "biological sample", refers to a biological material isolated from a subject. The biological sample contains any material suitable for detecting the sequence of a gene and can be a material comprising genetic material from the subject. The biological sample may comprise cellular and/or non-cellular material from the subject. In one embodiment, the sample comprises non-cellular material. In another embodiment, the sample comprises cellular material. In another preferred embodiment, the samples comprises a mixture of cellular and non-cellular material. In a particular embodiment, the sample comprises genetic material, for example, DNA, genomic DNA (gDNA), complementary DNA (cDNA), RNA, mRNA, etc., from the subject under study. In a particular embodiment, the genetic material is RNA. In a preferred embodiment, the genetic material is DNA. In a more preferred embodiment, the DNA is genomic DNA. The sample can be isolated from any biological tissue or fluid, such as, for example, blood, saliva, plasma, serum, urine, cerebrospinal fluid (CSF), feces, nasal, buccal, or buccopharyngeal swabs, a specimen, a specimen obtained from a biopsy, and a paraffin-embedded tissue sample. The methods for isolating samples are well known to those skilled in the art. In a preferred embodiment, the sample is isolated from blood. In another preferred embodiment, the sample is isolated from saliva.

Before analyzing the sample, it will often be desirable to perform one or more operations for preparing said sample to separate the molecule to be determined from other molecules found in the sample. In a particular embodiment, the molecules are nucleic acids, DNA, and/or RNA. In a preferred embodiment, the molecules are DNA. These operations for preparing the samples include manipulations such as: concentration, suspension, extraction of intracellular material (for example, nucleic acids of whole tissue/cell samples and the like), nucleic acid amplification, fragmentation, transcription, labeling, and/or extension reactions. These methods are well known to one skilled in the art. There are also commercial kits available for mRNA purification including, without limitation, miRNeasy Mini kit from Qiagen, miRNA isolation kits from Life Technologies, the mirPremier microRNA isolation kit from Sigma-Aldrich, and the High Pure miRNA isolation kit from Roche. In a particular embodiment, RNA integrity was analyzed using RNA 6000 Nano Chips in the Agilent 2100 bioanalyzer (Agilent Technologies, Palo Alto, CA, USA).

In a particular embodiment, the biological sample is a sample containing cells from an atherosclerotic plaque, more particularly a carotid atherosclerotic plaque. One skilled in the art knows the techniques for detecting atherosclerotic plaques, such as calculating the ankle-brachial index, magnetic resonance arteriogram, duplex ultrasound, and the hemodynamics for localizing purposes, or contrast arteriogram. Methods for the obtention of the atheroma plaque are well known in the art. In another embodiment, the cells extracted from the atherosclerotic plaque are endothelial cells. In another particular embodiment, the cells extracted from the atherosclerotic plaque are macrophages. In a preferred embodiment, the sample is a lysate of entire plaques, and therefore contains material derived from all the cells forming part of the plaque. In a more preferred embodiment, the cells extracted from the atherosclerotic plaque are smooth muscle cells (SMCs). Methods for the isolation of SMCs from the atherosclerotic plaque are well known in the art. Moreover, markers suitable for the identification of SMCs are also well-known in the art. In a particular embodiment, the SMCs are positive for the specific marker MYH11 and/or negative for any one, or any combination, of the specific markers PECAM1, CXCL9, CXCL10 and/or CD5L. In a more particular embodiment, the SMCs are MYH11-positive and PECAM1, CXCL9, CXCL10 and CD5L -negative. Various techniques for determining if a cell is positive or negative for a specific marker are known.

In a preferred embodiment, the determination of the SNP having accession number rs35286811 is carried out using a sample derived from an entire atherosclerotic plaque and it therefore contains genetic material derived from all the cell types of said plaque.

In a more preferred embodiment, the determination of the SNP having accession number rs35286811 is carried out using a sample derived from blood and/or from saliva.

### Method for determining the probability of a subject suffering from a cerebrovascular disease

The presence of an unstable atherosclerotic plaque is associated with an increase in the risk of suffering from a cerebrovascular disease (Insull, W. Am. J. Med. 122 (1 suppl), S3-S14 (2009). Accordingly, the SNP having accession number rs35286811 also allows classifying subjects based on their risk of suffering from a cerebrovascular disease.

Therefore, in a second aspect the invention relates to a prognostic method for determining the probability that a subject develops a cerebrovascular disease which comprises determining the SNP having accession number rs35286811 in a biological sample from said subject, and predicting the probability that the subjects develops a cerebrovascular disease according to the sequence of said SNP.

As it is used herein in the context of predicting methods of the invention, the term "determining the probability" or "predicting" refers to the prediction of a subject having a cerebrovascular disease, or it refers to the prediction of the progression of the cerebrovascular disease in a subject. As one skilled in the art will understand, said determination is not usually correct for all (i.e., 100%) of the patients to be identified. However, the term requires being able to identify a significant part of the subjects. One skilled in the art can readily determine if a part is statistically significant using several well-known statistical evaluation tools, for example, the determination of confidence intervals, the determination of p-values, Student's t-test, Mann-Whitney test, etc. Details can be found in Dowdy and Wearden, Statistics for Research, John Wiley and Sons, New York 1983. Preferred confidence intervals are at least 90%, at least 95%, at least 97%, at least 98%, or at least 99%. The p-values are preferably 0.1, 0.05, 0.01, 0.005, or 0.0001. More preferably, at least 60%, at least 70%, at least 80%, or at least 90% of the subjects of a population can be suitably identified by the method of the present invention.

As it is used herein, the term "cerebrovascular disease" refers to a clinical syndrome caused by the alteration of the blood supply to the brain, characterized by rapidly developing signs of an alteration of the overall cerebral function which last for more than 24 hours or lead to death. A cerebrovascular disease is usually known as stroke, given that it is the most common cerebrovascular disease. Cerebrovascular events according to the invention include transient ischemic attack (TIA), stroke, carotid artery disease, and amaurosis fugax. In a preferred embodiment, the cerebrovascular disease is stroke.

As it is used herein, the term "transient ischemic attack" or TIA refers to an episode of neurological dysfunction caused by ischemia (loss of blood flow either in the brain, the spinal cord, or the retina) without acute infarction (dead tissue). TIAs have the same underlying cause as strokes: a rupture of cerebral blood flow (CBF), and they are often referred to as mini-strokes or mega-strokes.

The term "stroke," also known as cerebrovascular accident (CVA), cerebrovascular incident (CVI), ictus or apoplexy, is used herein to refer to the situation in which there is a lack of blood flow to the brain, giving rise to cell death. Ischemic stroke (also known as ischemic infarction) is the result of the lack of blood flow, as a consequence of an obstruction of a blood vessel that supplies blood flow to the brain. Hemorrhagic stroke (also known as intracerebral hemorrhage) is due to a hemorrhage. As a result of the stroke, the brain does not function properly. The signs and symptoms of stroke may include: inability to move or lack of feeling on one side of the body, comprehension or speaking issues, dizziness, loss of vision, among others. The signs and symptoms often appear immediately after the stroke has taken place. If the symptoms last for less than 24 hours, it is known as a transient ischemic attack (TIA).

The term "carotid artery disease," also known as "carotid artery stenosis" or "carotid stenosis," refers to a stricture or constriction of the inner surface (lumen) of the carotid artery, usually caused by atherosclerosis. Methods for determining carotid artery stenosis are known to one skilled in the art and include, without limitation, Doppler duplex ultrasound, arteriogram, computed axial tomography angiography (CTA), or magnetic resonance angiography (MRA).

As it is used herein, the term "amaurosis fugax" refers to a painless and transient monocular vision loss.

In a particular embodiment, the sample is a sample containing cells from the atherosclerotic plaque. In another particular embodiment, the cells extracted from the atherosclerotic plaque are endothelial cells. In another particular embodiment, the cells extracted from the atherosclerotic plaque are macrophages. In a preferred embodiment, the sample is a lysate of entire plaques, and therefore contains material derived from all the cells forming part of the plaque. In a more preferred embodiment, the cells extracted from the atherosclerotic plaque are smooth muscle cells (SMCs).

In a particular embodiment, the SMCs are positive for the specific marker MYH11 and/or negative for any one, or any combination, of the specific markers PECAM1, CXCL9, CXCL10 and/or CD5L. In a more particular embodiment, the SMCs are MYH11-positive and PECAM1, CXCL9, CXCL10 and CD5L -negative. The methods for obtaining and identifying the cells of interest have been described above and are likewise applicable to this method.

According to the prognostic method of the invention, the first step comprises determining the SNP having accession number rs35286811.

The methods for determining the SNP of the gene of interest have been described above in the context of the diagnostic method of the invention and are likewise applicable in the present method, including the limitations thereof.

In a preferred embodiment if the SNP rs35286811 is a C, then the subject shows a high probability of suffering from a cerebrovascular disease. In a more preferred embodiment if the SNP rs35286811 in any one of the alleles is a C, then the subject shows a high probability of suffering from a cerebrovascular disease. In a more preferred embodiment if the SNP rs35286811 in both alleles is a C, then the subject shows a high probability of suffering from a cerebrovascular disease.

As it is used herein, "high probability" refers to the situation in which the subject shows at least 5%, at least 10%, at least 20%, at least 30%, at least 40%, at least 50%, at least 60%, at least 70%, at least 80%, at least 90%, at least 100% probabilities of having a cerebrovascular disease over time.

As it is used herein, "low probability" refers to the situation in which the subject shows at least 5%, at least 10%, at least 20%, at least 30%, at least 40%, at least 50%, at least 60%, at least 70%, at least 80%, at least 90%, at least 100% probabilities of not having a cerebrovascular disease over time.

The terms and limitations described above in relation to the diagnostic method of the invention are likewise applicable to this aspect.

### Method of selecting a patient susceptible of treatment for removing or stabilizing the carotid atherosclerotic plaque

The presence of an unstable atherosclerotic plaque is associated with an increase in the risk of suffering from a cerebrovascular disease (Insull, W. Am. J. Med. 122 (1 suppl), S3-S14 (2009). Accordingly, the SNP having accession number rs35286811 also allows selecting patients having unstable atherosclerotic plaque for a treatment to remove or stabilize said plaques.

Therefore, in a third aspect the invention relates to a method for selecting a patient susceptible of being treated with a therapy for removing or stabilizing the carotid atherosclerotic plaque which comprises determining the SNP having accession number rs35286811 in a biological sample from said patient, and selecting the patient as susceptible to being treated for removing or stabilizing the carotid atherosclerotic plaque according to the sequence of said SNP.

As it is used herein, the term "selecting" refers to the action of choosing a subject to subject him or her to a preventive or curative treatment for carotid atherosclerotic plaque, and therefore being able to prevent a cerebrovascular disease.

The term "treatment" or "therapy" includes any process, action, application, therapy, or the like, wherein a subject, including a human being, is provided medical aid with the object of improving the subject's condition, directly or indirectly, or slowing the progression of a condition or disorder in the subject, or ameliorating at least one symptom of the disease or disorder under treatment.

The first step of the method for selecting a patient susceptible of being treated with a therapy for removing or stabilizing the carotid atherosclerotic plaque comprises determining the SNP having accession number rs35286811 in a biological sample from said subject.

The methods for determining the SNP having accession number rs35286811, have been described above in the context of the diagnostic method of the invention and are likewise applicable in the present method, including the limitations thereof.

In a preferred embodiment if the SNP rs35286811 is a C, then the subject is susceptible of being treated with a therapy for removing or stabilizing the carotid atherosclerotic. In a more preferred embodiment if the SNP rs35286811 in any one of the alleles is a C, then the subject is susceptible of being treated with a therapy for removing or stabilizing the carotid atherosclerotic. In a more preferred embodiment if the SNP rs35286811 in both alleles is a C, then the subject is susceptible of being treated with a therapy for removing or stabilizing the carotid atherosclerotic.

In a preferred embodiment, the therapy for removing the carotid atherosclerotic plaque is carotid endarterectomy. In another preferred embodiment, the treatment involves a stent placement.

As it is used herein, the term "carotid endarterectomy," or EAC, refers to a surgical procedure used to reduce the risk of stroke, to correct stenosis (stricture) in the common carotid artery or internal carotid artery. Endarterectomy involves removing material from inside an artery.

In a preferred embodiment, the treatment for stabilizing the carotid atherosclerotic plaque comprises one or more statins, one or more antiaggregants or a combination thereof.

As it is used herein, the term "statin" refers to a inhibitor of 3-hydroxy-3-methylglutaryl-coenzyme A (HMG-CoA) reductase, the enzyme that catalyzes the limiting step of cholesterol biosynthesis, and includes any known or newly synthesized or newly designed natural, synthetic, or semi-synthetic statin or molecules related to statins; as it is used herein, the term "molecules related" to statins, refers to those molecules with lipid-lowering (cholesterol-lowering or triglyceride-lowering) capacity. Non-limiting illustrative examples of statins which can be used in the context of the present invention include atorvastatin, cerivastatin, fluvastatin, lovastatin, mevastatin, a monacolin (e.g., monacolin M, monacolin J, monacolin N, monacolin L, monacolin X, etc.), pitavastatin (also called itavastatin), pravastatin, rosuvastatin, simvastatin, etc., as well as combinations of any two or more statins, for example, monacolins, for example, monacolin M, monacolin J, monacolin N, monacolin L, monacolin X, etc., or any other combination of any two or more statins. Some statins can be in closed form (lactone) or in open form (hydroxy acid). Hydroxy acids (open form) can be prepared from the corresponding lactones by conventional hydrolysis, for example, with sodium hydroxide in methanol, sodium hydroxide in tetrahydrofuran-water, and the like.

As it is used herein, "antiaggregant" or "antiplatelet drug" refers to a compound the main effect of which is to inhibit platelet aggregation and therefore the formation of thrombi or clots in arteries and veins. Non-limiting illustrative examples of antiaggregants are cyclooxygenase inhibitors, such as acetylsalicylic acid, sulfinpyrazone, triflusal, ditazol, indobufen; ADP receptor inhibitors such as ticlopidine, clopidogrel, prasugrel; glycoprotein IIa/IIIb or GPIIb-IIIa receptor antagonists, such as tigramin, eptifibatide, tirofiban, abciximab.

In a particular embodiment, the sample is a sample containing cells from the atherosclerotic plaque. In another particular embodiment, the cells extracted from the atherosclerotic plaque are endothelial cells. In another particular embodiment, the cells extracted from the atherosclerotic plaque are macrophages. In a preferred embodiment, the sample is a lysate of entire plaques, and therefore contains material derived from all the cells forming part of the plaque. In a more preferred embodiment, the cells extracted from the atherosclerotic plaque are smooth muscle cells (SMCs).

In a particular embodiment, the SMCs are positive for the specific marker MYH11 and/or negative for any one, or any combination, of the specific markers PECAM1, CXCL9, CXCL10 and/or CD5L. In a more particular embodiment, the SMCs are MYH11-positive and PECAM1, CXCL9, CXCL10 and CD5L -negative. The methods for obtaining and identifying the cells of interest have been described above and are likewise applicable to this method.

The terms and limitations described above in relation to the diagnostic method and the prognostic method of the invention are likewise applicable to this aspect.

### Kits of the invention

The present invention also contemplates the preparation of kits for use in accordance with the present invention.

Thus, in another aspect, the invention relates to a kit comprising reagents suitable for determining the allele in the sequence of the SNP having accession number rs35286811.

In the context of the present invention, "kit" is understood as a product containing the different reagents required for carrying out the methods of the invention packaged such that it allows being transported and stored. The materials suitable for the packaging of the components of the kit include glass, plastic (polyethylene, polypropylene, polycarbonate, and the like), bottles, vials, paper, sachets, and the like. Furthermore, the kits of the invention can contain instructions for the simultaneous, sequential, or separate use of the different components that are in the kit. Said instructions can be in the form of printed material or in the form of an electronic medium capable of storing instructions such that they can be read by a subject, such as electronic storage media (magnetic disks, tapes, and the like), optical media (CD-ROM, DVD), and the like. The media may additionally or alternatively contain Internet addresses providing said instructions.

Materials suitable for inclusion in an exemplary kit in accordance with the present invention comprise one or more of the following: gene specific PCR primer pairs (oligonucleotides) that anneal to DNA or cDNA sequence domains that flank the rs35286811 SNP; reagents capable of amplifying a specific sequence domain in either genomic DNA or cDNA without the requirement of performing PCR; reagents required to discriminate between the various possible alleles in the sequence domains amplified by PCR or non-PCR amplification (e.g., restriction endonucleases, oligonucleotide that anneal preferentially to one allele of the polymorphism, including those modified to contain enzymes or fluorescent chemical groups that amplify the signal from the oligonucleotide and make discrimination of alleles more robust); or reagents required to physically separate products derived from the various alleles (e.g. agarose or polyacrylamide and a buffer to be used in electrophoresis, HPLC columns, SSCP gels, formamide gels or a matrix support for MALDI-TOF).

In a particular embodiment, the reagents of the kit comprise DNA and/or RNA probes.

In a particular embodiment, the kit comprises reagents suitable for determining at least one of the alleles in the sequence of the SNP having accession number rs35286811. In another particular embodiment, the kit comprises reagents suitable for determining both alleles in the sequence of the SNP having accession number rs35286811.

Specifically contemplated are kits comprising two or more allele-specific oligonucleotides or oligonucleotide pairs, wherein each of the allele-specific oligonucleotide or oligonucleotide pair is directed to the rs35286811 SNP. It will be appreciated that in this context the term "directed to" means an oligonucleotide or oligonucleotide pair capable of identifying the allele present at the SNP. By illustrative, the present invention contemplates a kit comprising a probe set, comprising a plurality of oligonucleotide probes which make up at least 10%, at least 20%, at least 30%, at least 40%, at least 50%, at least 60%, at least 70%, at least 80% or at least 90% of the oligonucleotide probes in the probe set. In a preferred embodiment, the kit includes a set of two oligonucleotide probes, specific to one allele of the rs35286811 SNP.

In another embodiment, the kit according to the present invention further comprises reagents suitable for determining the expression levels of genes that are expressed specifically or are not expressed in smooth muscle cell populations, specifically MYH11, PECAM1, CXCL9, CXCL10 and/or CD5L.

In another aspect, the present invention relates to the use of a kit according to the invention for diagnosing unstable atherosclerotic plaques in a subject, for determining the probability of a subject suffering from a cerebrovascular disease, or for selecting a patient susceptible to being treated with a therapy for removing and/or stabilizing the carotid atherosclerotic plaque, based on the sequence of the rs35286811 SNP. The particulars of the kit according to the invention have been described in detail in the context of the kit of the invention and are applied with same meaning in the context of the uses of said kit.

The terms and limitations described above in relation to the diagnostic method, the prognostic method of the invention and the method for selecting a patient are likewise applicable to this aspect.

The invention will be described by way of the following examples which are to be considered as merely illustrative and not limitative of the scope of the invention.

### EXAMPLES

### Materials and methods

### Patient selection

Patients who underwent carotid endarterectomy at Basurto University Hospital were selected to be included in the current study on the bases of clinical parameters (age, symptoms, no other clinical manifestations). Degree of stenosis was evaluated with carotid cervical Eco-Doppler ultrasound and tomographic angiography according to established criteria. Symptomatic (Symp) patients were identified as those with >70% stenosis and presenting symptoms of transient ischemic attack or ipsilateral stroke within the past 6 months, while asymptomatic (Asymp) patients were those with stenosis >80% without any presence of cerebrovascular disease. All patients were evaluated before and after the operation by a specialized neuropsychologist. Brain MRI was performed in all patients before and after the surgery to monitor potential complications. Only patients who fulfil all the required parameters were finally included in the study: 7 Asymp vs 7 Symp for RNAseq analysis and 89 Asymp vs 104 Symp. Carotid tissue sample was collected after surgery and transported immediately to the lab for cell isolation and RNA extraction.

### Cell isolation

Fresh carotid artery tissue samples from symptomatic and asymptomatic patients were processed and cut in 2-3 mm size fragments, which were digested with collagenase type I (Life Technologies, Carlsbad, CA, USA) to further isolate and culture SMCs. First, a 3 hours digestion was performed at 300U/mL concentration of collagenase type I. Second, digested tissue was submitted to overnight digestion with 220U/mL collagenase type I. Digested tissue was filtered by a 100 µm nylon Falcon™ Cell Strainer for removing undigested material, and cells were plated in specific medium for human SMC growth called M231 (Life Technologies, Carlsbad, CA, USA) supplemented SMCs growth factors such us 20 ng/ml IGF-1, 2 ng/ml EGFB, 0,5 ng/ml EGF, 5ng/ml heparin, 0,2 µg/ml BSA and 5% newborn calf serum (Sigma, St Louis, MO, USA). Second digestion yielded higher amount of cells. RNA used for RNAseq was extracted from cells from passage 0. RNA used for quality control analysis was extracted from SMCs isolated after 3 or 16 hours collagenase digestion and from passages 0 and 1. Quality control of pure SMCs culture was performed by identification of the presence of SMC-specific marker (MYH11) and absence of endothelial marker (PECAM1) and macrophage markers (CXCL9, CXCL10 and CD5L). RNA extracted from monocytic cell line U937 induced with LPS (100 ng/ml) during 6 hours and CD14+ monocyte-derived dendritic cells treated with LPS (100 ng/ml) for 6 hours were used respectively as positive controls for CXCL9 and CXCL10 it was used.

### RNA extraction from SMCs

Extraction and purification of RNA from SMCs was performed with TRIzolTM reagent according to manufacture's instructions. RNA was then purified with RNeasy Total RNA Isolation Kit (QIAGEN, Valencia, CA, USA) following the instructions recommended by the manufacturer. RNA integrity (RIN>9) was analysed by using RNA 6000 Nano Chips on the Bioanalyzer Agilent 2100 (Agilent Technologies, Palo Alto, CA, USA).

### RNA library assembly

Ribosomal RNA removal was performed with the Ribo-Zero rRNA kit removal kit. Generation of libraries was performed with the TruSeq Stranded Total RNA library Prep kit following manufacture's recommendations. 2 µg of total RNA (RIN>9) libraries were used as a start, which were sequenced using a HiSeq2000 instrument (Illumina Inc, San Diego, CA, USA). Sequencing readings were paired-end with a length of about 100 bp reading performed in 14 samples. The estimated coverage was around 60 million reads per sample (3 lanes). Library generation and cDNA sequencing was done at Sistemas Genómicos S.L. (Valencia, Spain) following manufacturer's instructions.

### Data analysis

The quality control of the raw data was performed using the FastQC tool. Then, the raw paired-end reads were mapped against the human genome provided by Ensembl database (version GRchr37/hg19) using tophat2 algorithm (Kim, D. et al. 2013. TopHat2: accurate alignment of transcriptomes in the presence of insertions, deletions and gene fusions. Genome Biol. 14). Insufficient quality reads (phred score <10) were eliminated using the Picard Tools software (version 1.129). In this step, the GC distribution was assessed (i.e. the proportion of guanine and cytosine bp along the reads), which should have a desired distribution between 40-60%. Second, distribution of duplicates (quality of sequencing indicator) were evaluated to confirm that our sequencing contained small proportion of duplicates. Gene predictions were estimated using the Cufflinks method [2] and the expression levels were calculated using the HT Seq software (version 0.6.0, http://www-huber.embl.de/users/anders/HTSeq/). This method employs unique reads for the estimation of gene expression and eliminates the multimapped reads.

For variant calling, GATK RNAseq best practices guidelines were followed. GATK v3.7 was employed to add read groups, mark duplicates, Split'N'Trim, reassing mapping qualities, indel realignment, base recalibration, variant calling and filtering. The variants annotation process was realized using the variant effect predictor of ensembl. The eQTL process with cis and trans relationship were process using seqGene algorithm. The association was selected on the basis of with a pValue without adjust threshold of 0.10.

### DNA extraction from full plaque

Carotid atherosclerotic tissue from 290 samples was used to extract genomic DNA with NYZ Tissue gDNA isolation kit following manufacturer's instructions. DNA was quantified using the Qubit Fluorometer to determine the concentration and quality ratios (A260/280 and A260/230)

### Genotyping

Fluidigm SNP genotyping was performed according to the manufacturer's instructions using the Fluidigm 48.48 dynamic genotyping array. It was used SNPType genotyping assays designed for SNPs identified in previously disclosed sequencing analysis. Briefly, STA (specific targe amplification) reaction was performed with specific primers. The diluted amplified product was loaded into the 48.48 Dynamic Array IFC (integrated fluidic circuits) for SNP genotyping together with a sample mixture, a ROX reference dye and real-time master mix. Endpoint fluorescence images of the 48.48 IFC were acquired on an EP1 Fluidigm imager and the data was analyzed with Fluidigm Genotyping Analysis Software. The differences in genotype and allele frequencies of all SNPs in symptomatic and asymptomatic were analyzed by chi-square (x2)

### RESULTS

### Example 1

### Identification of SNPs associated with carotid atherosclerosis symptomatology

RNA isolated from symptomatic (7 samples) and asymptomatic (7 samples) was used for RNAseq analysis. A total of 1.005.934.806 reads from the 14 sequenced samples were obtained with an average of 77.852.486 reads per sample. Reads were aligned against the human genome using the tophat2 algorithm. The percentage of mapped reads ranged from 82,2% to 87,6% per sample. The GATK tool was used to call variants. More than 240 SNPs associated with symptomatology (42 novel and 208 previously reported), identified with GATK, were annotated with variant effect predictor (Ensembl) with p adjusted value < 0,05. 36 SNPs in 16 genes with significant p value (p<0.05) for eQTLcis were identified with SeqGen algorith. 12 SNPs in 12 of these genes were further selected on the basis of significance and potential association with phenotype (Table 1).

**Table 1. SNPs selected for validation analysis.**

| **Gene Symbol** | **eQTL pvalue** | **Phenotype pvalue** | **SNP** |
|---|---|---|---|
| PDLIM4 | 0,027 | 0,02 | rs9895 |
| ATL3 | 0,038 | 0,02 | rs79429913 |
| BIRC6 | 0,07 | 0,02 | rs35286811 |
| TMEM167A | 0,05 | 0,02 | rs13162274 |
| HTT | - | 0,05 | rs363075 |
| BDH2 | - | 0,02 | rs6825519 |
| SLK | - | 0,02 | rs10883960 |
| NEDD4 | - | 0,02 | rs2899593 |
| MGLL | - | 0,004 | rs76232599 |
| RMND1 | - | 0,004 | rs1065310 |

### Example 2

### SNP validation

A total of 12 SNPs including 4 with eQTL p value <0.08 were selected for further validation by digital PCR using the Fluidigm platform. SNP assays were run on a total of 290 samples using 96.96 IFC Dynamic Arrays and the SNPtype assays. 1 SNP (rs35286821, BIRC6) out of the 12 successfully genotype variants was associated with symptomatology. Genotype and allele frequencies of the 12 SNPs in symptomatic and asymptomatic are represented in Table 2. Data thus indicate that rs35286822 is a genomic marker that can be used to distinguish asymptomatic from symptomatic patients, with the C allele associated with enhanced risk for symptomatology.

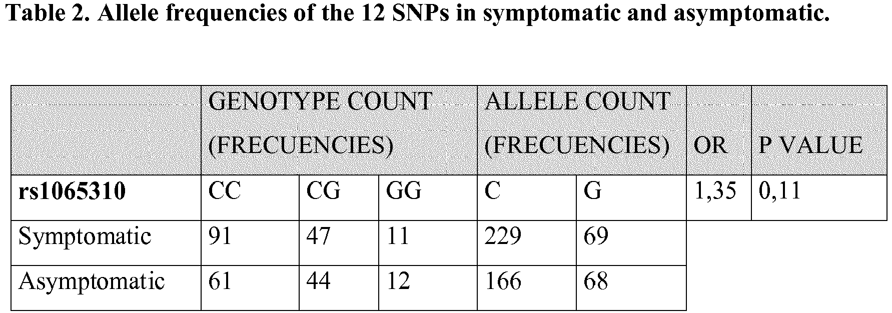

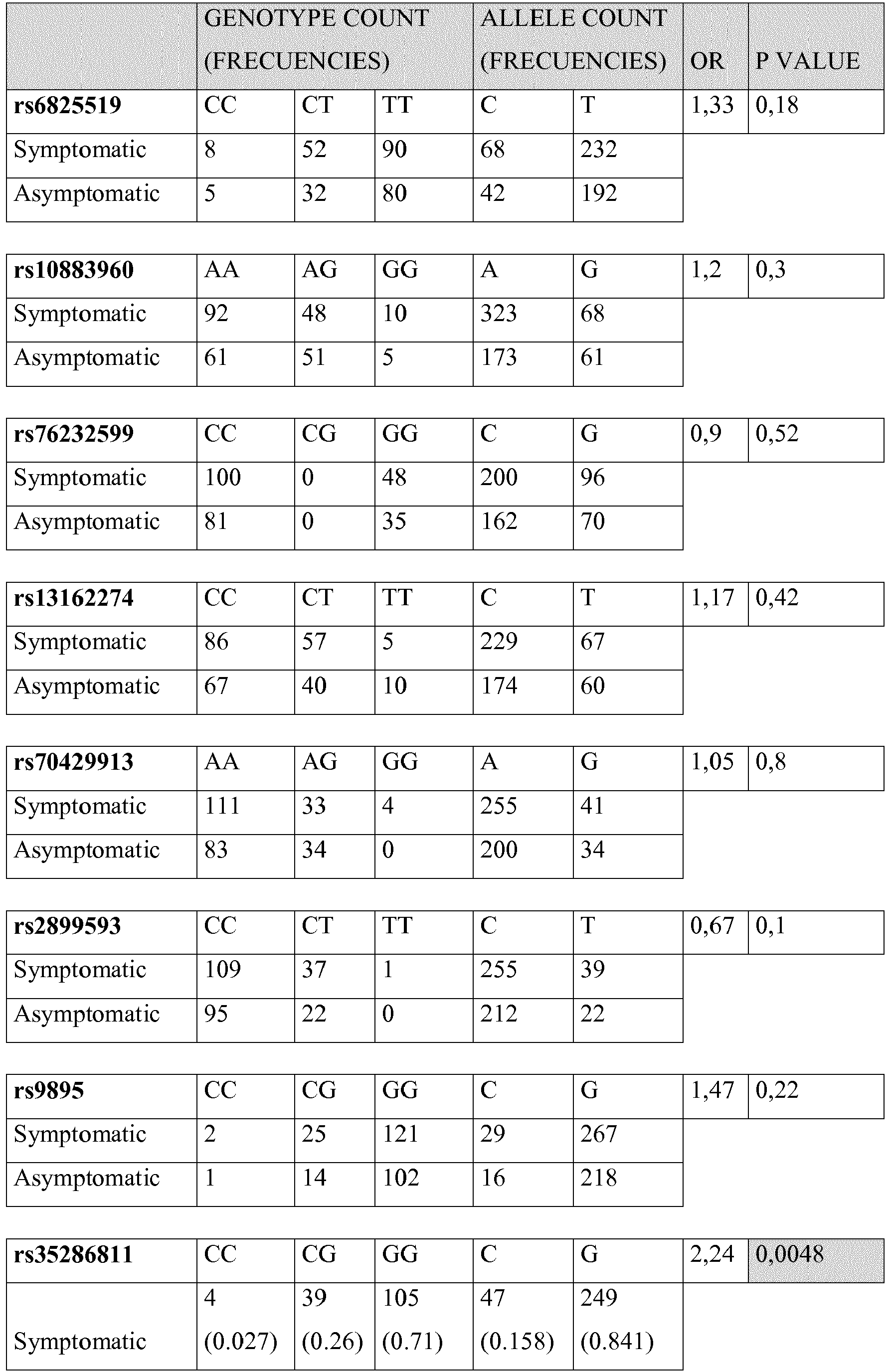

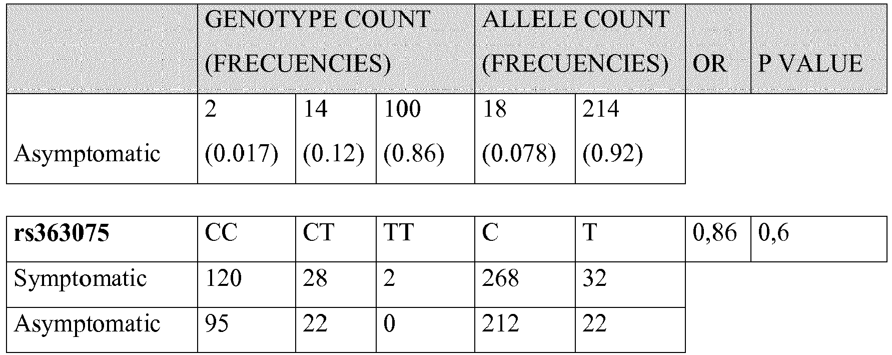

## Claims

1. A method for diagnosing unstable atherosclerotic plaques in a subject which comprises determining the SNP having accession number rs35286811 in a biological sample from said subject, and diagnosing unstable atherosclerotic plaque in the subject according to the sequence of said SNP.

2. The method according to claim 1 wherein if the SNP is a C, then the subject is diagnosed with unstable atherosclerotic plaque.

3. The method according to any one of claims 1 or 2, wherein the biological sample is a sample of blood and/or saliva.

4. A prognostic method for determining the probability that a subject develops a cerebrovascular disease which comprises determining the SNP having accession number rs35286811 in a biological sample from said subject, and predicting the probability that the subject develops a cerebrovascular disease according to the sequence of said SNP.

5. The method according to claim 4 wherein if the SNP is C, then the subject has a high probability of developing a cerebrovascular disease.

6. The method according to any one of claims 4 or 5, wherein the biological sample is a sample of blood and/or saliva.

7. The method according to any one of claims 4 to 6 wherein the cerebrovascular disease is stroke.

8. A method for selecting a patient susceptible of being treated with a therapy for removing or stabilizing the carotid atherosclerotic plaque which comprises determining the SNP having accession number rs35286811 in a biological sample from said patient, and selecting the patient as susceptible to being treated for removing or stabilizing the carotid atherosclerotic plaque according to the sequence of said SNP.

9. The method according to claim 8, wherein if the SNP is C, then the patient is susceptible to being treated with a therapy for removing or stabilizing the carotid atherosclerotic plaque.

10. The method according to any one of claims 8 or 9, wherein the therapy for removing the carotid atherosclerotic plaque is carotid endarterectomy or the therapy for stabilizing the plaque comprises one or more statins, one or more antiaggregants or a combination thereof.

11. The method according to any one of claims 8 to 10, wherein the biological sample is a sample of blood and/or saliva.

12. A kit comprising reagents suitable for determining the allele in the sequence of the SNP having accession number rs35286811.

13. Use of a kit according to claim 13 for diagnosing unstable atherosclerotic plaques in a subject, for determining the probability of a subject suffering from a cerebrovascular disease, or for selecting a patient susceptible to being treated with a therapy for removing or stabilizing the carotid atherosclerotic plaque.
